# EUROPÄISCHE PATENTSCHRIFT

(11) **EP 0 946 593 B1**
(45) Veröffentlichungstag und Bekanntmachung des Hinweises auf die Patenterteilung: **20.07.2005**
(21) Anmeldenummer: 97954370.9
(22) Anmeldetag: 10.12.1997
(51) Int. Cl.: C07K 14/52, A61K 38/00

(54) **VERWENDUNG VON HCC-1 ZUR BEHANDLUNG VON THROMBOZYTOPENIEN**
USE OF HCC-1 FOR TREATING THROMBOCYTOPENIA
UTILISATION D' HCC-1 POUR LE TRAITEMENT DE LA THROMBOCYTOPENIE

(30) Priorität: 13.12.1996 EP 96120024
(43) Veröffentlichungstag der Anmeldung: 06.10.1999
(73) Patentinhaber: Pharis Biotec GmbH, 30625 Hannover (DE)
(72) Erfinder: OPITZ, Hans-Georg, D-69469 Weinheim (DE); SCHMITT, Joachim, D-68519 Viernheim (DE); FORSSMANN, Wolf-Georg, D-30625 Hannover (DE); SCHULZ-KNAPPE, Peter, D-30966 Hemmingen (DE)
(74) Vertreter: Meyers, Hans-Wilhelm, Dr.Dipl.-Chem.
(86) Internationale Anmeldenummer: PCT/EP1997/006881
(87) Internationale Veröffentlichungsnummer: WO 1998/025966

(56) Entgegenhaltungen:
- WO-A-93/09799
- WO-A-94/24285
- WO-A-94/28916
- WO-A-95/17092
- WO-A-96/19234
- WO-A-97/06817
- S. D. WOLPE ET AL: "Macrophage inflammatory proteins 1 and 2: members of a novel superfamily of cytokines" THE FASEB JOURNAL, Bd. 3, Dezember 1989, Seiten 2565-2573, XP000606882 in der Anmeldung erwähnt
- B.I. LORD ET AL: "Macrophage inflammatory protein: its characteristics, biological properties and role in the regulation of haemopoiesis" INTERNATIONAL JOURNAL OF HEMATOLOGY, Bd. 57, 1993, Seiten 197-206, XP000611694 in der Anmeldung erwähnt
- M. G. HUNTER ET AL: "BB-10010: An Active Variant of Human Macrophage Inflammatory Protein-1alpha with Improved Pharmaceutical Properties" BLOOD, Bd. 86, Nr. 12, 15.Dezember 1995, Seiten 4400-4408, XP000611695 in der Anmeldung erwähnt

## Beschreibung

Die Erfindung betrifft die Verwendung des Polypeptides HCC1, aus der Klasse der MIPs (macrophage inflammatory proteins) zur Behandlung von Erkrankungen, die mit einer pathologischen Verminderung der Blutplättchenbildung einhergehen.

Thrombozyten spielen eine zentrale Rolle bei der Blutgerinnung und der Wundheilung. Thrombozytenerkrankungen können somit zu Störungen in der hämostatischen Blutstillung und damit zu massiven Blutungen führen. Man unterscheidet generell Thrombozytopenien, bei denen die Anzahl der Thrombozyten erniedrigt ist, und Thrombozytendysfunktionen, bei denen trotz normaler Thrombozytenzahlen deren Funktion gestört ist.

Als Ursachen einer Thrombozytopenie kommen verminderte oder fehlende Megakaryozyten im Knochenmark, verminderte Plättchenproduktion, Sequestration der Thrombozyten in der Milz, vermehrter Thrombozytenabbau, vermehrter Thrombozytenverbrauch oder Verdünnung des Thrombozytenpools in Frage. Gleich welcher Ätiologie, führt eine schwere Thrombozytopenie zu Hautblutungen in Form von multiplen Petechien, die meist an den Unterschenkeln am deutlichsten hervortreten, sowie zu verstreuten kleinen Ekchymosen nach Bagatelltraumen. Gravierender sind jedoch Schleimhautblutungen (Nasenbluten; Blutungen im Gastrointestinaltrakt, im Urogenitaltrakt und in der Vagina) sowie Blutungen nach chirurgischen Eingriffen. Abhängig vom Ausmaß der Thrombozytopenie können schwere gastrointestinale Blutungen sowie Blutungen in das Zentralnervensystem mit lebensbedrohlichen Folgen auftreten.

Thrombozytopenien können bei einer Vielzahl von Erkrankungen und bei bestimmten therapeutischen Eingriffen beobachtet werden. Eine verminderte Plättchenbildung kann auftreten in Folge, z.B. einer myelosuppressiven Therapie (Bestrahlung, Chemotherapie insbesondere Hochdosistherapie), einer Knochenmarktransplantation, einer Leukämie, einer Anämie (aplastische Anämie, Fanconi Anämie etc.) oder auch nach Alkoholabusus. Einem vermehrten Thrombozytenverbrauch oder -abbau können Erkrankungen zugrunde liegen, wie Idiopathische Thrombozytopenische Purpura, Infektionen (Thrombozytopenie bei HIV-Infektion, Sepsis etc.), immunologische Grunderkrankungen (Autoimmunerkrankungen wie z.B. Systemischer Lupus Erythematodes), chronische Leukämie oder maligne Lymphome. Eine verminderte Plättchenzahl kann auch als medikamenteninduzierte Thrombozytopenie nach Gabe von beispielsweise Heparin, Chinidin, Sulfonamiden, oralen Antidiabetika, Goldsalzen oder Rifampicin auftreten.

Im Rahmen der Diagnostik in der klinischen Chemie ist die Bestimmung der Zellzahlen im peripheren Blutbild der wesentliche Parameter, um das Ausmaß der Thrombozytopenie festzustellen, und um erste Hinweise auf mögliche Ursachen zu erhalten. So weist z.B. ein vermehrter Anteil an großen Thrombozyten (bestimmbar anhand des Blutausstriches oder durch Messung des mittleren Thrombozytenvolumens in einem elektronischen Blutzellanalysegerät) auf eine kompensatorisch erhöhte Thrombozytenproduktion hin. Diese findet man oft bei sekundären Thrombozytopenien aufgrund eines erhöhten Abbaus oder Verbrauchs der Thrombozyten. Die Blutungszeit ist bei schweren Thrombozytopenien jeglicher Ursache verlängert. Bei Patienten mit nur mäßiger Thrombozytopenie. (z.B. 50000 Thrombozyten/µl) kann die Bestimmung der Blutungszeit jedoch eine wertvolle Information sein. Eine stark verlängerte Blutungszeit weist in dem Fall darauf hin, daß eine Beladung der Thrombozyten mit Antikörpern offensichtlich zu einer Funktionsstörung der zirkulierenden Thrombozyten führt. Ein weiteres diagnostisches Kriterium stellen die Untersuchungen von Knochemarkpunktionen dar. Die Thrombozyptopenien können dabei nach Anzahl und Erscheinungsbild der Megakaryozten beurteilt werden.

Zur Behandlung von Thrombozytopenien müssen die auslösenden Ursachen erkannt und korrigiert werden, z.B. bei medikamenten-induzierten Thrombozytopenien durch rasches Absetzen des die Thrombozytopenie auslösenden Medikamentes oder durch Erkennung und Behandlung einer Infektion mit endotoxinbildenden gram-negativen Keimen. Wenn die Thrombozytopenie Folge einer Störung der Megakaryopoese und damit verminderten Produktion von Plättchen nach z.B. einer Chemotherapie ist, kann durch Gabe von Thrombozytenkonzentraten die Thrombozytenzahl meist für die Dauer von 2-3 Tagen angehoben werden. Zur Behandlung der Thrombozytopenie werden Thrombozytenkonzentrate entweder kontinuierlich (1 bis 2 Einheiten pro Stunde) oder in größeren Mengen im Abstand von einigen Stunden gegeben, z.B. 6 bis 8 Einheiten alle 4 bis 6 Stunden. Prophylaktisch sollten Thrombozytenkonzentrate jedoch mit Zurückhaltung eingesetzt werden, da wegen der Entwicklung von Thrombozyten-Alloantikörpern ihre Effektivität bei wiederholter Anwendung nachlassen kann. Auch darf bei Transfusionen das Risiko einer Infektion nicht vernachlässigt werden. Wenn eine rasche Regeneration der Knochenmarkfunktion nicht zu erwarten ist, sollte die Thrombozytentransfusion nur für die Behandlung einer klinisch manifesten Blutungsneigung eingesetzt werden. Bei Thrombozytopenien infolge vermehrten Plättchenverbrauchs sollten Thrombozytentransfusionen nur in Ausnahmefallen prophylaktisch gegeben werden, da bei diesen Erkrankungen die Thrombozyten innerhalb einer bis weniger Stunden wieder aus dem Kreislauf eliminiert werden. Bei der Behandlung der heparininduzierten Thrombozytopenie können transfundierte Thrombozyten auch zur Bildung von Thrombozyten-Fibrin-Thromben und so zu einer schweren Thromboseneigung führen.

Polypeptide aus der Klasse der MIPs (macrophage inflammatory proteins) sind bekannt aus WO 95/17092. Diese Polypeptide werden beispielsweise von Makrophagen oder Lymphozyten sezerniert, wenn diese durch gram-negative Bakterien, Lipopolysaccharide oder Concanavalin A stimuliert werden. WO 95/17092 beschreibt derartige Polypeptide und deren Verwendung zur Herstellung von Arzneimitteln zur Behandlung von Infektionen, Krebs, Entzündungen, myelopoetischen Dysfunktionen oder Autoimmunerkrankungen. Insbesondere wird in Zusammenhang mit der Hämatopoese die Hemmung von Knochenmarkstammzellen durch MIPs beschrieben, um beispielsweise myeloproliferative Erkrankungen zu behandeln. Die hemmende Wirkung von MIPs auf die Stammzellproliferation wird auch als Therapieprinzip in der Krebsbehandlung beschrieben, um Stammzellen durch vorhergehende Verabreichung von MIPs ruhigzustellen und so vor den Nebenwirkungen einer Chemotherapie zu schützen. Darüberhinaus beschreibt WO 95/17092 Inhibitoren bzw. Antagonisten gegen derartige Polypeptide, die eine von MIPs verursachte Knochenmarkssuppression aufheben sollen und damit zur Behandlung von z.B. der aplastischen Anämie oder des myelodysplastischen Syndroms eingesetzt werden können. Eine Behandlung der Thrombozytose durch Gabe von MIPs soll durch Erhöhen der Gefäßpermeabilität in der Peripherie und damit vermehrter Sequestration von Thrombozyten erreicht werden. In einem kürzlich erschienenen Meetingabstract wird für das aus WO 95/17092 bekannte MIP1γ beschrieben, daß die Reifung der Vorläuferzellen von Monozyten/Makrophagen in Gegenwart von MIP1γ inhibiert wird. MIP1γ wird demzufolge als M-CIF (Macrophage colony inhibition factor) bezeichnet.

Ferner ist aus DE 43 44 397 ein weiteres MIP-Fragment mit der Bezeichnung HCC-1 bekannt. Bei diesem Fragment handelt es sich um ein Polypeptid, das die Sequenz von MIP-3 (1-69) umfaßt und N-terminal um fünf weitere Aminosäuren verlängert ist. Dieses Peptid findet therapeutisch Verwendung zur Behandlung von Störungen der Migration von Zellen, Erkrankungen des Immunsystems, Tumoren sowie Dysfunktion regulatorischer Wachstumsfaktoren.

Aus WO 97/06871 ist bekannt, daß durch die kontinuierliche Verabreichung von Stammzell-Chemokinen ("SCC" = stem cell chemokins) eine verstärkte Wiederherstellung des haematopoetischen Systems im Zusammenhang mit der myelosuppressiven Therapie erzielt werden kann. Als Stammzell-Chemokine wird dort beispielsweise auf die Proteine LD78 (huMIP-1α), muMIP-1α, MIP-1β, IL-8, GRO, NAP-2, MCAF, ENA78, PF4, GCP-2, INPROL, MCP-1, MCP-2 und MCP-3 und deren Analoga verwiesen. Die Wiederherstellung des haematopoetischen Systems wird als zufriedenstellend definiert, wenn die Werte an Neutrophilen, Plättchen und/oder an Vorläuferzellen wieder auf einen Bereich von mehr als 25 % des Normalwertes ansteigen. Die Verabreichung der Stammzell-Chemokine wird jedoch nur im Zusammenhang mit der myelosuppressiven Therapie (MT), insbesondere vor Beginn der MT, während der MT und direkt nach der MT beschrieben.

In WO 96/19234 wird die kombinierte Verwendung von CxC Chemokinen und von Haematopoese stimulierenden Mitteln, wie z.B. CSFs, zur Erhöhung der Freisetzung und Mobilisierung von haematopoetischen Zellen aus dem Knochenmark beschrieben. Solche Kombinationspräparate werden eingesetzt bei Behandlungen, bei denen die Anreicherung von Blut mit haematopoetischen Stammzellen therapeutisch sinnvoll ist, z.B. bei peripheren Blutstammzell-Transplantationen und zur Erhöhung der Immunantwort.

In WO 94/28916 wird die Verwendung von Stammzell-Inhibitoren ("SCI"), wie z.B. muMIP-1α oder huMIP-1α und deren Analoga zur Verstärkung der Freisetzung und Mobilisierung von haematopoetischen Stammzellen beschrieben. Die Stammzell-Inhibitoren werden beispielsweise verwendet bei der Behandlung von Neutropenien, die im Zusammenhang mit der Chemotherapie auftreten.

Überraschenderweise wurde nun gefunden, daß das Polypeptid HCC-1 aus der Klasse der MIPs und dessen Derivate eine signifikante und spezifische Erhöhung der Blutplättchenzahl bewirken und somit zur Herstellung von Arzneimitteln zur Behandlung von Krankheitszuständen, die allgemein mit einer Verringerung der Thrombozytenzahlen einhergehen, verwendet werden können. Dies umfaßt vor allem solche Erkrankungen, die entweder mit einer Störungen der Megakaryopoese oder einer pathologischen Verminderung der Blutplättchenbildung einhergehen. In diesem Zusammenhang wird dieses Polypeptid insbesondere zur Behandlung von Thrombopenien verwendet, wie beispielsweise Thrombopenien, die durch Chemotherapie oder Bestrahlung ausgelöst wurden; oder Thrombopenien, die im Zusammenhang mit Knochenmarktransplantationen, viralen oder retroviralen Infektionen, Autoimmunerkrankungen und Anämien stehen. Die therapeutische Wirkung dieses Polypeptides wurde in vivo durch Stimulation der Thrombopoese nachgewiesen. Dabei konnte nach einer vorhergehenden Behandlung mit Chemotherapeutika ein schnelleres Erholen der Vorläuferzellen im Knochenmark und damit eine deutlich verkürzte Dauer des thrombopenischen Zustandes erreicht werden.

Als Derivate von HCC-1 kommen auch solche Polypeptide in Frage, die durch Verlängerung, Deletion oder Substitution einzelner oder mehrerer Aminosäuren abgeleitet werden (vgl. beispielsweise WO 94/28916; WO 95/17092; WO 93/09799; WO 94/24285; The Faseb Journal 1989, 3: 2565-2573; Int. J. Hematol. 1993, 57: 197-206; Blood 1995, 86(12): 4400-4408). Derartige Fragmente oder Derivate sind beispielsweise solche Polypeptide, in denen eine oder mehrere Aminosäuren durch natürliche oder andere Aminosäuren ersetzt werden, eine oder mehrere der Aminosäuren einen Substituenten aufweisen oder das Polypeptid an andere Verbindungen (wie z.B. Polyethylenglykol) gekoppelt werden, um beispielsweise die Halbwertszeit zu erhöhen. Ferner kann das Polypeptid N- und/oder C-terminal um eine oder mehrere Aminosäuren verlängert oder verkürzt werden, wobei jedoch im wesentlichen die biologische Aktivität des Polypeptides im Hinblick auf die Stimulation der Thrombopoese erhalten bleibt. Entsprechende Verlängerungen sind insbesondere Leadersequenzen oder sekretorische Sequenzen, wobei bis zu 40 Aminosäuren, bevorzugt bis zu 20 oder 10 Aminosäuren N-terminal der Sequenz des reifen Polypeptids vorausgehen können.

Die biologische Wirksamkeit des erfindungsgemäßen Peptids wird beispielhaft anhand folgender Untersuchungen beschrieben:

### Beispiel

Stimulation der Thrombopoese durch HCC-1 nach Busulfan-Behandlung von Balb/C Mäusen

### Material und Methoden:

Balb/C Mäusen (weiblich, 6-8 Wochen alt, 17 g Körpergewicht) wird einmalig am Tag 0 Busulfan in einer Dosierung von 20 mg/kg intraperitoneal appliziert. Als Lösungsvermittler für Busulfan dient Cremophor EL. Beginnend mit Tag 1 bis Tag 15 wird den Tieren täglich einmal HCC-1 in PBS/1% BSA als Lösungsmittel intraperitoneal verabreicht. Die applizierten Dosen liegen zwischen 0.01 und 10 µg/Maus. Kontrolltiere erhalten nur Lösungsmittel PBS/1% BSA einmal täglich i.p.. Zu den angegebenen Zeitpunkten wird den Tieren 20 µl Vollblut retrobulbär entnommen und die verschiedenen Blutparameter in einem Blutanalysator (Contraves Autolyzer 801) bestimmt.

### Ergebnis.

Nach Application des Zytostatikums Busulfan wird in Mäusen bei geeigneter Dosierung eine vorübergehende Thrombozytopenie induziert (Morley und Blake, Oyekan und Onabanjo). Der Verlauf der Plättchenzahlen im peripheren Blut von Balb/C Mäusen nach Einmalgabe von Busulfan 20 mg/kg wird zeitabhängig aufgetragen. Aus der graphischen Darstellung ist zu erkennen, daß beginnend mit Tag 8 nach der Injektion die Plättchenzahlen in der Busulfankontrolle abfallen und ihren Nadir bereits zwischen Tag 11 und 13 erreichen. Die Reduktion der PLT-Werte beträgt dabei etwa 60%. Bis Tag 21 steigen die Plättchenzahlen dann wieder an, ein Reboundphänomen ist nicht zu beobachten. Bis Tag 35 bleiben die PLT-Werte in der Busulfangruppe unterhalb der unbehandelten Kontrolle und erreichen das Ausgangsniveau nicht mehr.

Nach Busulfangabe und anschließender täglicher Application von HCC-1 kommt es ebenfalls ab Tag 8 zu einem Abfall der Plättchenzahlen. Die erreichten Werte für den Nadir liegen jedoch bei allen Dosierungen von HCC-1 oberhalb der Busulfankontrolle. Darüberhinaus wird der Zeitraum bis zum Wiederanstieg der Plättchenzahlen deutlich verkürzt, so daß in der Gruppe mit HCC-1 1 µg/Maus bereits am Tag 15 wieder PLT-Werte um 800 x 10⁶/ml gemessen werden können. Im weiteren Verlauf des Experiments liegen die PLT-Zahlen in den HCC-1 behandelten Gruppen oberhalb der Busulfankontrolle und erreichen etwa ab Tag 30 wieder das Niveau von unbehandelten Kontrolltieren. Legt man als Grenze 700 x 10⁶ PLT/ml fest, so wird die Dauer und das Ausmaß der Thrombopenie (gemessen als Fläche unter der Kurve) durch die Applikation von HCC- 1 im Vergleich zur Busulfankontrolle um bis zu 70% reduziert.

**Tabelle 1**

| Einfluß von HCC-1 auf das Ausmaß der Thrombopenie in Busulfan-behandelten Balb/C-Mäusen | | | | |
|---|---|---|---|---|
| **Busulfan** | **Therapie** | **Dosis** | **Thrombopenie**^{**a**} | |
| | | **[pro Maus]** | **[%]** | **% Reduktion** |
| + | PBS-BSA | - | 100 | - |
| + | HCC-1 | 10 µg | 45 | 55 |
| + | HCC-1 | 1 µg | 30 | 70 |
| + | HCC-1 | 0,1 µg | 35 | 65 |
| + | HCC-1 | 0,01 µg | 58 | 42 |

| | | | | |
|---|---|---|---|---|
| ^{a} = der Beginn der Thrombopenie wurde auf 700x10⁶ PLT/ml festgelegt und die Fläche unterhalb der Kurve für jede Therapiegruppe ermittelt (n = 8 Tiere/Gruppe) | | | | |

## Patentansprüche

1. Verwendung des Polypeptides HCC-1 zur Herstellung von Arzneimitteln zur Behandlung von Erkrankungen, die mit einer pathologischen Verminderung der Blutplättchenbildung einhergehen.

2. Verwendung nach Anspruch 1 zur Behandlung von Erkrankungen, die mit einer Senkung der Megakaryopoese einhergehen.

3. Verwendung nach Anspruch 1 oder 2 zur Behandlung von Thrombopenien.

4. Verwendung nach Anspruch 3 zur Behandlung von Thrombopenien, die durch Chemotherapie oder Bestrahlung ausgelöst wurden.

5. Verwendung nach Anspruch 3 zur Behandlung von Thrombopenien, die im Zusammenhang mit Knochenmarktransplantationen, viralen oder retroviralen Infektionen, Autoimmunerkrankungen und Anämien stehen.

6. Verwendung nach einem der Ansprüche 1- 5, **dadurch gekennzeichnet, daß** das Polypeptid HCC-1 gekoppeltan Polyethylen glykol ist.

## Claims

1. Use of the polypeptide HCC-1 for the preparation of medicaments for treating diseases involving a pathological reduction of platelet formation.

2. The use according to claim 1 for treating diseases involving a reduction of megakaryopoiesis.

3. The use according to claim 1 or 2 for treating thrombocytopenia.

4. The use according to claim 3 for treating thrombocytopenia induced by a chemotherapy or radiotherapy.

5. The use according to claim 3 for treating thrombocytopenia related to bone marrow transplantations, viral or retroviral infections, autoimmune diseases and anemia.

6. The use according to any of claims 1 to 5, **characterized in that** said polypeptide is HCC-1 coupled to polyethylene glycol.

## Revendications

1. Utilisation du polypeptide HCC-1 pour préparer des médicaments destinés au traitement de maladies qui sont associées à une réduction pathologique de la formation de plaquettes sanguines.

2. Utilisation selon la revendication 1 pour le traitement de maladies qui sont associées à une diminution de la mégacaryocytopoïèse.

3. Utilisation selon la revendication 1 ou 2 pour le traitement de thrombopénies.

4. Utilisation selon la revendication 3 pour le traitement des thrombopénies qui sont déclenchées par la chimiothérapie ou l'exposition à des radiations.

5. Utilisation selon la revendication 3 pour le traitement des thrombopénies qui sont liées à des greffes de la moelle osseuse, des infections virales ou rétrovirales, des maladies auto-immunes et des anémies.

6. Utilisation selon une des revendications 1 à 5, **caractérisée en ce que** le polypeptide HCC-1 est lié à du polyéthylène glycol.
